# EUROPÄISCHE PATENTANMELDUNG

(11) **EP 0 873 722 A1**
(43) Veröffentlichungstag der Anmeldung: **28.10.1998**
(21) Anmeldenummer: 97810252.3
(22) Anmeldetag: 24.04.1997
(51) Int. Cl.: A61B 17/36

(54) **Gerät für eine endokardiologische Behandlung**

(71) Anmelder: Sulzer Osypka GmbH, 79639 Grenzach-Wyhlen (DE)
(72) Erfinder: Geistert, Wolfgang, Dr., 79618 Rheinfelden-Herten (DE); Kitschmann, Achim, 79639 Grenzach-Wyhlen (DE)
(74) Vertreter: Heubeck, Bernhard

(57) **Zusammenfassung**

Das Gerät für eine kardiologische Therapie, insbesondere ein Ablationsgerät umfasst einen Ultraschall-Transducer (1) und einen bei dem Transducer angeordneten, zu einer endokardialen Energieabgabe vorgesehenen Applikator (7). Mittels des Transducers ist eine echokardiographische Dickenmessung des Myokards (M) durchführbar. Es sind Steuerungsmittel vorgesehen, mit denen die Energieabgabe mit dem Applikator dosiert und auf die gemessene Dicke abgestimmt vornehmbar ist.

## Beschreibung

Die Erfindung betrifft ein Gerät für eine kardiologische Therapie, insbesondere ein Ablationsgerät, gemäss Oberbegriff von Anspruch 1.

Ablationsgeräte dienen dazu, mittels endokardialen Eingriffen Herzrhythmusstörungen zu beseitigen. Mit bekannten Geräten wird das Herzgewebe lokal soweit erhitzt, dass lediglich die elektrischen Eigenschaften des Gewebes geschädigt werden. Als Energiequelle für die Ablation wird oft ein Hochfrequenz-Generator verwendet (z.B. Osypka HAT200S, HAT300, Medtronic Atakr, EPT 1000). Ultraschall und Mikrowelle können ebenfalls als Energiequellen benutzt werden. Ferner ist eine Ablation auch durch Unterkühlen oder mittels chemischer Stoffe durchführbar. Diese beiden Einwirkmöglichkeiten sollen hier in Verbindung mit dem Begriff "Energieabgabe" mitumfasst sein.

Mit Vorteil wird die Energieabgabe mittels am Applikator durchgeführter Temperaturmessung geregelt. Diese Regelung dient dazu, eine Koagulation von Gewebe sowie von Blut und damit Trombenbildungen zu verhindern.

Die WO 95/17131 beschreibt einen Katheter und ein Verfahren, bei dem mittels Ultraschall die Lage des Katheters zum Gewebe bestimmbar ist. Das Ziel ist dabei, die Energie nur dann abzugeben, wenn der Katheter in geeigneter Ausrichtung zum Gewebe ist und in unmittelbarem Kontakt mit ihm steht. Die Energie lässt sich so mit gutem Wirkungsgrad in das Gewebe einbringen; eine unnötige und schädliche Abgabe von Energie an das Blut wird dabei vermieden.

Wie bekannt ist, kann mittels Ultraschall die Dicke des Myokards echokardiographisch gemessen werden (siehe z.B. J.H. Myers et al "Direct Measurement of Inner and Outer Wall Thickening Dynamics with Epicardial Echocardiography", Circulation, Vol.74, No.1, July 1986, Seiten 164 ff). Gewebeabmessungen, also insbesondere Myokarddicken, können mit den meisten bildgebenden Ultraschallgeräten bestimmt werden.

Bei häufigen Herzrhythmusstörungen wie beispielsweise Vorhofflimmern ist es wichtig, dass die elektrischen Eigenschaften des Myokards in die Tiefe gehend durchgängig zerstört werden, so dass in dem behandelten Gewebe keine Erregungsleitung mehr stattfinden kann. Besonders bei der Beseitigung von Flimmern, das sich in chaotischen Erregungen äussert, ist es in vielen Fällen schwierig, den Ablationserfolg auf elektrischem Weg zu kontrollieren.

Es ist daher Aufgabe der Erfindung, ein Gerät für eine kardiologische Therapie, insbesondere ein Ablationsgerät, zu schaffen, bei dem mit grosser Sicherheit das zu behandelnde Gewebe nach der Energieabgabe tatsächlich durchgehend denaturiert ist. Es sollen dabei nur die elektrischen Eigenschaften des Gewebes verändert werden, und zwar gerade soweit, dass Herzrhythmusstörungen anschliessend nicht mehr auftreten.

Diese Aufgabe ist mit dem in Anspruch 1 definierten Gerät lösbar. Mit diesem Gerät ist eine Dosierung der Energieabgabe möglich. Mittels Ultraschall wird die Dicke des Myokards bestimmt; aufgrund der gemessenen Dicke lässt sich die Abgabe der therapeutischen Energie steuern. Die Energie kann mit dem erfindungsgemässen Gerät gezielt so dosiert werden, dass sich eine durchgehende Denaturierung des Gewebes ergibt, ohne dass dabei gleichzeitig Schichten, die in einer grösseren Entfernung vom Applikator liegen, irreparabel geschädigt werden.

Das erfindungsgemässe Gerät für eine kardiologische Therapie, insbesondere ein Ablationsgerät, umfasst einen Ultraschall-Transducer und einen bei dem Transducer angeordneten, zu einer endokardialen Energieabgabe vorgesehenen Applikator. Mittels des Transducers ist eine echokardiographische Dickenmessung des Myokards durchführbar. Es sind Steuerungsmittel vorgesehen, mit denen die Energieabgabe mit dem Applikator dosiert und auf die gemessene Dicke abgestimmt vornehmbar ist.

Die abhängigen Ansprüche 2 bis 10 beziehen sich auf vorteilhafte Ausführungsformen des erfindungsgemässen Geräts.

Nachfolgend wird die Erfindung anhand der Zeichnungen erläutert. Es zeigen:
- Fig. 1: ein Blockschaltbild des erfindungsgemässen Geräts,
- Fig. 2: das distale Ende eines Katheters zum erfindungsgemässen Gerät,
- Fig. 3: das distale Ende eines Katheters gemäss einer zweiten Ausführungsform,
- Fig. 4: eine dritte Ausführungsform und
- Fig. 5: ein Element, in dem Ultraschall-Transducer und Applikator eine Einheit bilden.

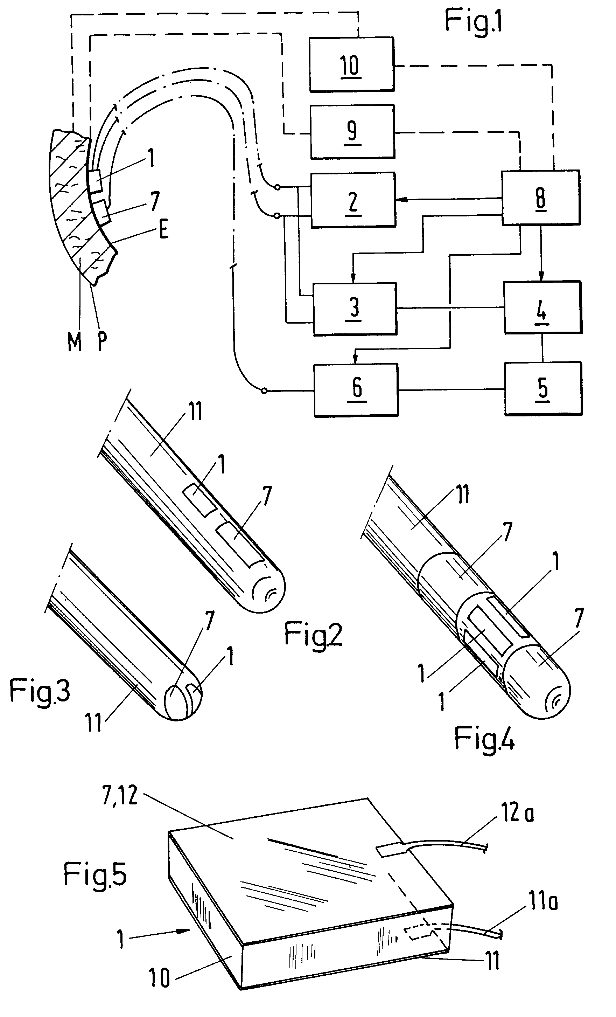

Folgende Komponenten werden von dem Gerät gemäss Blockschaltbild der Fig.1 umfasst: ein Ultraschall-Transducer 1, ein Pulserzeuger 2, ein Verstärker 3, ein Signalanalysator 4, ein Dosierer 5, eine Energiequelle 6, ein Applikator 7 und eine Zeitablaufsteuerung 8. Ferner - lediglich bei besonderen Ausführungsformen - eine Einrichtung 9, welche die Herzexpansion und -kontraktion wahrnimmt, und/oder ein Herzschrittmacher 10. Der Transducer 1 und der Applikator 7 sind am Myokard M angeordnet.

Dem Transducer 1 wird ein im Pulserzeuger 2 generierter elektrischer Impuls zugeleitet, dort in Schallenergie umgewandelt und abgestrahlt. Danach wirkt der Transducer 1 als Empfänger: Er nimmt von den Gewebestrukturen reflektierte Schallwellen auf und wandelt sie in elektrische Energie um. Diese Energie wird nach Durchlauf des Verstärkers 3 und nach einer gegebenenfalls vorgesehenen Demodulierung dem Signalanalysator 4 zugeführt. Dort werden aus einer Amplitudenmodulation von Hüllkurven die Reflexionen, die von Endokard E und Perikard P herstammen, ermittelt. Aus den Zeitpunkten, zu denen die Reflexionen beim Transducer eintreffen, und aus der bekannten Schallgeschwindigkeit im Gewebe lässt sich die Dicke des Myokards M berechnen.

Der so festgestellte Wert der Myokarddicke wird dem Dosierer 5 zugeleitet. Mittels eines auf die Energiequelle bezogenen Rechenalgorithmus oder mittels einer empirisch aufgestellten Datenbank wird die therapeutische Energie bestimmt, mit der die elektrischen Eigenschaften des Gewebes zerstörbar sind. Der Dosierer 5 leitet die Information über die notwendige Energie an die Energiequelle 6 weiter, welche anschliessend automatisch oder jeweils nach einer Aufforderung eine entsprechende Energiemenge über den Applikator 7 an den Myokard M abgibt.

Die Zeitablaufsteuerung 8 koordiniert den korrekten zeitlichen Ablauf des Verfahrens, der mit Vorteil auf die Herzbewegung abgestimmt ist.

In bevorzugten Ausführungsformen der Erfindung sind der Ultraschall-Transducer 1 und der Applikator 7 im gleichen Katheter 11 angeordnet: siehe Figuren 2 bis 4.

Es ist von Vorteil, wenn Ultraschall-Transducer 1 und Applikator 7 so ausgerichtet sind, dass die Energie nur in die Richtung abgegeben wird, in der die Dicke gemessen worden ist.

Ferner ist es von Vorteil, die in Fig.1 angegebene Einrichtung 9 vorzusehen, welche die Herzkontraktion und -expansion wahrnimmt. Es kann so der Zeitpunkt der Dickenmessung mit einem vom Herz kommenden Signal synchronisiert werden. Somit liegen bei der Messung stets die gleichen reproduzierbaren Verhältnisse vor.

Die Synchronisierung lässt sich wesentlich erleichtern, wenn das Herz mit einem Herzschrittmacher 10 (Fig.1) periodisch stimuliert wird und so eine konstante Periodizität der Herzbewegung erzwungen wird. Mit dem Signal des Herzschrittmachers 10 lässt sich auch der Zeitpunkt der Dickenmessung steuern.

Bei dem Ausführungsbeispiel der Fig.2 sind der Transducer 1 und der Applikator 7 seitlich am distalen Ende des Katheters 11 angeordnet. Beim zweiten Ausführungsbeispiel, Fig.3, sind der Transducer 1 und der Applikator 7 an der distalen Spitze des Katheters 11 angeordnet. Das dritte Ausführungsbeispiel, Fig.4, zeigt einen Katheter 11 mit zwei Applikatoren 7 und einer Mehrzahl von Ultraschall-Transducern 1.

In einer besonders vorteilhaften Ausführungsform sind Transducer 1 und Applikator 7 in einem gemeinsamen Element integriert: Fig.5. Ein Ultraschall-Transducer 1 umfasst beispielsweise einen plättchenförmigen Piezokristall 10, dessen beiden Seiten je eine Elektrode 11, 12 tragen. Dieser Transducer ist so in einem Katheter angeordnet (nicht gezeigt), dass sich die eine Elektrode 12 unmittelbar an der Oberfläche des Katheters befindet. Diese Elektrode 12 lässt sich in einer Weise ausbilden, dass sie als Applikator 7 verwendbar ist. Ein Draht 11a stellt eine elektrische Verbindung zur Elektrode 11 her. Eine zweite elektrische Verbindung 12a bildet den Anschluss an die Elektrode 12 bzw. den Applikator 7. Über die Verbindung 12a wird zusätzlich die für die kardiologische Therapie vorgesehene Energie übertragen.

## Patentansprüche

1. Gerät für eine kardiologische Therapie, insbesondere ein Ablationsgerät, mit einem Ultraschall-Transducer (1) und einem bei dem Transducer angeordneten, zu einer endokardialen Energieabgabe vorgesehenen Applikator (7),
dadurch gekennzeichnet, dass mittels des Transducers eine echokardiographische Dickenmessung des Myokards (M) durchführbar ist und dass Steuerungsmittel vorgesehen sind, mit denen die Energieabgabe mit dem Applikator dosiert und auf die gemessene Dicke abgestimmt vornehmbar ist.

2. Gerät nach Anspruch 1, dadurch gekennzeichnet, dass die endokardiale Energieabgabe mittels eines Systems durchführbar ist, das zusätzlich zu Transducer (1) und Applikator (7) folgende Komponenten umfasst: einen Pulserzeuger (2), einen Verstärker (3), einen Signalanalysator (4), eine Energiequelle (6), einen Dosierer (5) und eine Zeitablaufsteuerung (8), wobei der Pulserzeuger - zwecks Übertragung von elektrischen Impulsen - mit dem Transducer verbunden ist und dieser - zwecks Übertragung von im Transducer durch reflektierte Schallwellen erzeugten Signalen - mit dem Verstärker verbunden ist.

3. Gerät nach Anspruch 1 oder 2, dadurch gekennzeichnet, dass eine an das Gerät angeschlossene Einrichtung (9) vorgesehen ist, mit welcher die Herzkontraktion und -expansion registrierbar ist.

4. Gerät nach einem der Ansprüche 1 bis 3, dadurch gekennzeichnet, dass ein Anschluss an einen Herzschrittmacher (10) vorgesehen ist, mit dem aufgrund einer periodischen Stimulierung eine konstante Periodizität der Herzbewegung erzwingbar ist, wobei mit dem Signal des Herzschrittmachers (10) der Zeitpunkt der Dickenmessung steuerbar ist.

5. Gerät nach einem der Ansprüche 1 bis 4, dadurch gekennzeichnet, dass der Ultraschall-Transducer (1) und der Applikator (7) benachbart im distalen Bereich eines Katheters (11) angeordnet sind.

6. Gerät nach Anspruch 5, dadurch gekennzeichnet, dass der Transducer (1) und der Applikator (7) so ausgerichtet sind, dass die Abgaberichtung für die Energie mit der Richtung übereinstimmt, aus welcher der Transducer Echosignale für die Dickenmessung empfängt.

7. Gerät nach einem der Ansprüche 5 oder 6, dadurch gekennzeichnet, dass der Ultraschall-Transducer (1) und der Applikator (7) in einem gemeinsamen Element integriert sind.

8. Gerät nach einem der Ansprüche 5 bis 7, dadurch gekennzeichnet, dass der Transducer (1) und der Applikator (7) seitlich am Katheter (11) angeordnet sind.

9. Gerät nach einem der Ansprüche 5 bis 7, dadurch gekennzeichnet, dass der Transducer (1) und der Applikator (7) an der distalen Spitze des Katheters (11) angeordnet sind.

10. Gerät nach einem der Ansprüche 5 bis 9, dadurch gekennzeichnet, dass eine Mehrzahl von Ultraschall-Transducern (1) und/oder Applikatoren (7) im distalen Bereich des Katheters (11) vorgesehen sind.
